# EUROPÄISCHE PATENTSCHRIFT

(11) **EP 0 866 080 B1**
(45) Veröffentlichungstag und Bekanntmachung des Hinweises auf die Patenterteilung: **31.07.2002**
(21) Anmeldenummer: 98103485.3
(22) Anmeldetag: 27.02.1998
(51) Int. Cl.: C08F 246/00, A61L 27/00, A61L 29/00, A61L 33/00, C08F 214/06

(54) **Formmassen mit antikoagulierenden Eigenschaften, ihre Herstellung und Verarbeitung zu Gegenständen, die in der Medizintechnik Verwendungen Finden**
Mouldings with anticoagulant properties, their preparation and their use in the manufacturing of articles useful in medical fields
Masses de moulage avec des propriétés anticoagulantes, leur préparation et leur usage en articles formés utilisables dans la technique médicale

(30) Priorität: 01.03.1997 DE 19708426; 26.09.1997 DE 19742528; 18.02.1998 DE 19806748
(43) Veröffentlichungstag der Anmeldung: 23.09.1998
(73) Patentinhaber: Vestolit GmbH & Co.KG, 45764 Marl (DE)
(72) Erfinder: Jozefowicz, Marcel, Prof., 60260 Lamorlaye (FR); Migonney, Véronique, Dr., 95600 Eaubonne (FR); Meyer, Heinz Hermann, Dr., 45770 Marl (DE); Neu, Thomas, Dr., 48249 Dülmen (DE); Stieneker, Axel, Dr., 48151 Münster (DE); Anders, Christine, Dr., 45721 Haltern (DE); Ottersbach, Peter, Dr., 51570 Windeck (DE)
(74) Vertreter: Sternagel, Fleischer, Godemeyer & Partner Patentanwälte

(56) Entgegenhaltungen:
- EP-A- 0 093 489
- EP-A- 0 444 232
- US-A- 3 841 903
- US-A- 5 057 566
- CHEMICAL ABSTRACTS, vol. 122, no. 10, 6. März 1995 Columbus, Ohio, US; abstract no. 115027, XP002106639 & JP 06 206935 A (M.AKASHI ET AL.) 26. Juli 1994
- PATENT ABSTRACTS OF JAPAN vol. 017, no. 700 (C-1145), 21. Dezember 1993 & JP 05 237181 A (NIPPON FINE CHEM CO LTD;OTHERS: 01), 17. September 1993

## Beschreibung

Die Erfindung betrifft die Herstellung von Formmassen mit bestimmten bioaktiven Funktionen, spezielle Maßnahmen bei der Verarbeitung dieser Formmassen zu Gegenständen mit oberflächenwirksamen bioaktiven Funktionen sowie die Verwendung dieser Gegenstände in der Medizintechnik, insbesondere im Kontakt mit Blut oder seinen Bestandteilen.

Der Begriff «Bioaktivität» soll hier im weitesten Sinn als Wechselwirkung zwischen bestimmten statistisch angeordneten Funktionen oder Kombinationen solcher Funktionen in synthetisch erzeugten Makromolekülen und komplementären Funktionen natürlicher makromolekularer Systeme verstanden werden.

Die Wechselwirkung zwischen Blut und fremden Oberflächen, beispielsweise Polymeroberflächen, ist weitgehend bekannt; der Kontakt' bestimmter im Blut enthaltener Proteine mit diesen fremden Oberflächen führt zu einer kaskadenartigen Aktivierung verschiedener biochemischer Folgereaktionen, die in der sogenannten Komplementaktivierung oder der Blutkoagulation gipfeln. Seit den 50er Jahren wurde in einer Fülle von detaillierten Experimenten der Versuch unternommen, die Wechselwirkung zwischen fremden Oberflächen und Blut so zu beeinflussen, daß bei vorubergehendem oder auf langzeitigem Kontakt die Blutkoagulation sowie nachteilige Reaktionen des Immunsystems wie Entzündungen etc. unterbleiben.

Die Palette der Maßnahmen ist weit gespannt und teilweise widersprüchlich, wie die folgende Übersicht zeigt, vgl. R. Barbucci u a. «Modifications to Polymer Surfaces to Imporve Blood Compatibility», S. 119 ff. in. Polymeric Biomaterials, S. Dumitriu Herausg., Marcel Dekker Inc., 1994:
◆ Einstellung hydrophober oder hydrophiler Oberflächen
   - hydrophobe Oberflächen nach Alkylierung, dadurch bevorzugte Adsorption von Albumin, dadurch Reduzierung der Blutkoagulation
   - Hydrophilierung von Polymeroberflächen mit Polyethylenglykolketten, dadurch verminderte Protein- und Blutplättchenadsorption
◆ Erzeugung von Oberflächen mit Zwitterionen- oder Anionenstruktur
   - neutrale Zwitterionenstrukturen, z.B. polymergebundene Phosphorylcholin-Kopfgruppen (EP 0 275 295, 1986, A.A. Durrani)
   - anionische Strukturen bestimmter Konzentration und Zusammensetzung

Die erfindungsgemäßen Maßnahmen folgen dieser letztgenannten Entwicklungsrichtung.

Hier wurde längere Zeit das natürlich vorkommende Heparin, ein Glykosaminglykan mit Carboxyl-, Sulfat- und Aminsulfatgruppen, favorisiert. Es wurde adsorptiv, ionisch oder kovalent an Polymeroberflächen gebunden; man stellte jedoch fest, daß seine Aktivität relativ schnell abklingt und somit die für klinische Langzeitanwendungen erforderliche Stabilität nicht gegeben ist. Eine weitere Entwicklung bestand darin, die als bioaktiv erkannten Funktionen des Heparins, nämlich Carboxylat und Sulfat/Sulfonat, durch naßchemische polymeranaloge Umsetzungen an den Oberflächen einer Auswahl von vernetzten Polymeren oder an gelösten Polymeren zu fixieren, um auf diese Weise ein heparinähnliches Verhalten zu erzeugen.

Hier sind insbesondere die Arbeiten von Jozefowicz, Jozefowicz und Mitarbeitern zu nennen, vgl. EP-A 0 023 854, 0 090 100, 0 094 893, 0 201 378, 0 203 865 und 0 304 377.

Auch mit Hilfe von Energiequellen wie ionisierenden Strahlen oder elektrischen Entladungen. können Polymeroberflächen in der oben beschriebenen Weise modifiziert werden, vgl. R. Barbucci u.a. in obigem Zitat, S. 204 ff.

Schließlich besteht auch die Möglichkeit, die bioaktiven an Vinylmonomere gebundenen Funktionen durch Copolymerisation in Polymersysteme einzuführen oder in dünner Polymerschicht an Polymeroberflächen zu binden.

Beispielhaft für diese Entwicklungsrichtung sind Arbeiten von Miller, Sawyer u.a., J. Appl. Polym. Sci. 1970, 14, 257-266, von Sorm, Nespurek u.a., J. Polym. Sci., Polym. Symp. 1979, 66, 349-356 sowie die EP 0 598 486 der MEDTRONIC Inc. vom 14.10.93. In dieser neueren Anmeldung werden Kombinationen der monomeren Acrylsäure und 2-Acrylamido-2-methylpropansulfonsäure, insbesondere im Molverhältnis 2 : 7, beansprucht. Dieses Monomergemisch wird entweder für sich copolymerisiert und anschließend auf Polymeroberflächen aufgetragen, z. B. durch Tauchen in entsprechenden Lösungen; oder es wird in Gegenwart von Cer-Ionen direkt auf Polymeroberflächen gepfropft.

Allen genannten Verfahren, sofern sie sich auf feste Polymere und deren Oberflächen beziehen, ist gemeinsam, daß die bioaktiven Funktionen in einem weiteren Verarbeitungsschritt auf bereits ausgeformten Gegenständen fixiert werden. Die Nachteile solcher Verfahren liegen auf der Hand:
◆ Die in der Humanmedizin unabdingbare, reproduzierbare Einstellung identischer bioaktiver Oberflächen, z.B. für eine Serienfertigung von Kathetern und Blutbeuteln, ist auf diese Weise nicht zu gewährleisten.
◆ Die nachträgliche Modifizierung ist aufwendig bezüglich Zeit und Materialaufwand (Ökonomie und Ökologie).
◆ Die Wiederverwendbarkeit (Recycling) solcher oberflächenmodifizierter Gegenstände ist nicht möglich, weil nach einem erneuten Umschmelz- und Verarbeitungsschritt nicht die gleichen bioaktiven Oberflächen vorliegen werden.

Diese Nachteile konnten nun durch die Verwendung von Formmassen gemäß den Patentansprüchen überwunden werden.

Gegenstand der Erfindung ist die Verwendung von Formmassen mit antikoagulierenden Eigenschaften, wobei hydrophobe Monomere mit hydrophilen, mit biospezifisch wirksamen Funktionen ausgestatteten Monomeren radikalisch copolymerisiert werden, in der Medizintechnik.
Es wurde überraschend gefunden, daß durch radikalische Copolymerisation einer bestimmten Auswahl geeigneter Vinylmonomerer Formmassen hergestellt werden können, die von Polymerisatteilchen zu Polymerisatteilchen chemisch homogen mit bioaktiven Funktionen versehen sind und die je nach Konzentration und Kombination der mit den Vinylmonomeren eingebrachten bioaktiven Funktion zu Gegenständen ausgeformt werden können, die vielseitige Verwendung in der Medizintechnik finden.

Darüber hinaus wurde gefunden, daß die Verarbeitung zu Gegenständen mit oberflächenwirksamen bioaktiven Funktionen besondere Maßnahmen erfordert, um diese bioaktiven Funktionen auch nach der Verarbeitung gleichmäßig über die Innen- und Oberflächenbereiche der Gegenstände zu verteilen oder gar an der Oberfläche anzureichern.

Die Herstellung der erfindungsgemäß verwendeten Formmassen mit antikoagulierenden Eigenschaften zeichnet sich dadurch aus, daß hydrophobe Monomere mit hydrophilen und biospezifisch wirksamen Funktionen ausgestatteten Monomeren radikalisch copolymerisiert werden.

Ein besonders vielseitiges Verfahren zur Herstellung der erfindungsgemäßen Formmassen ist die radikalische Copolymerisation. Diese kann in Masse, in Lösung mit anschließender Fällung oder in Dispersion erfolgen. Die Copolymerisation in Dispersion umfaßt alle bekannten Verfahren, bei denen das Monomerengemisch oder Teile desselben in disperser Form in einem flüssigen, rührbaren Medium vorliegen. Dieses Trägermedium ist in der Regel Wasser, kann aber auch eine organische Phase sein. Zu diesen bekannten Verfahren zählen die Suspensionspolymerisation und die Emulsionspolymerisation mit in Wasser dispergierter organischer Phase, aber auch ihre inversen Ausführungsformen, bei denen die wäßrige Phase mit den wasserlöslichen Monomeren in organischer Phase dispergiert ist. Auch die speziellen Techniken der Mikro- und Mini-Emulsionspolymerisation können grundsätzlich zur Herstellung der beanspruchten Formmassen angewendet werden. Des weiteren ist auch eine Lösungs-/Fällungscopolymerisation von Vinylchlorid in Alkylalkoholen wie z. B. Methanol, Ethanol, Propanol oder Isopropanol möglich.

Da die Löslichkeiten und Reaktivitäten der verwendbaren Monomeren recht unterschiedlich sein können, müssen die Polymerisationsreaktionen prozeßrechnerkontrolliert und -gesteuert so ausgeführt werden, daß der Einbau der bioaktiven Funktionen möglichst gleichrnäßig in allen Makromolekülen und vollständig erfolgt. Deshalb wird von den an sich bekannten Techniken der dosierten Zugabe von Monomerbausteinen während der Polymerisation (Zulaufverfahren) und von der Polymerisation in Stufen Gebrauch gemacht, wie das später in den Beispielen deutlich gemacht wird.

Die Auswahl der radikalisch copolymerisierbaren Monomeren umfaßt sowohl hydrophobe als auch hydrophile, in der Regel mit bioaktiven Funktionen ausgestattete Monomere. Zu den verwendeten hydrophoben Monomeren zählen vor allem Styrol, Methylmethacrylat und Vinylchlorid. Unter den hydrophilen Monomeren werden solche verwendet, die bioaktive Grundfunktionen wie Hydroxyl-, Carboxyl- und Sulfatyl- bzw Sulfonylgruppen tragen. Dazu zählen die Hydroxyalkyl(meth)acrylate und Mono-(EO)ₙ-Vinylether und -Vinylester, Acrylsäure und Methacrylsäure sowie Maleinsäure und Maleinsäureanhydrid und die davon abgeleiteten Halbester und schließlich Vinylsulfonate im weitesten Sinn, vor allem Vinylarylsulfonate wie Styrolsulfonat.

Darüber hinaus können weitere Monomere eingesetzt werden, die als Umsetzungsprodukte der genannten carboxyl- und sulfonylhaltigen Vinylmonomere mit Aminosäuren oder Purin- bzw. Pyrimidinbasen angesehen werden können. Sie sind aufgrund ihrer Struktur in der Lage, spezifische Bindungen zu Proteinen, Enzymen und lebenden Zellen auszubilden.

Bevorzugt werden die Monomeren in Dispersionen radikalisch copolymerisiert.

Die biospezifisch wirksamen Funktionen der hydrophilen Monomeren sind ganz oder teilweise mit weiteren biospezifisch wirksamen Funktionen zu

M-F₁

M-F₂

M-F₃

M-F₁-F₄

wobei
- M =: Rest des hydrophilen Monomeren und
- F₁ =: Alkyl-, Aryl- oder Alkyl/Arylhydroxyl-
- F₂ =: Alkyl-, Aryl- oder Alkyl/Arylcarboxyl-
- F₃ =: Alkyl-, Aryl- oder Alkyl/Arylsulfonyl-
- F₄ =: Alkyl-, Aryl- oder Alkyl/Arylphosphatyl-
substituiert.

Die biospezifisch wirksamen Funktionen der Formmassen können auch wie folgt aussehen:

M - F₂ - S₁

M - F₃ - S₁

M - F₁ - F₄ - S₁

M - F₁ - F₄ - S₂

M - F₁ - F₄ - S₃

Mit S₁ = Rest einer Aminosäure, die über -NH₂ an Carboxyl-, Sulfonyl- oder Phosphatyl- gebunden ist, wobei die bevorzugten Aminosäure Glutamin, Threonin, Methionin, Cystein, Cysteinsäure, Prolin, Hydroxyprolin, Serin, Tyrosin, Benzoyloxycarbonyl-Lysin, tert.-Butyloxycarbonyl-Lysin, ε-Aminocapronsäure, β-Alanin, γ-Amino-n-Buttersäure und substituierte oder unsubstituierte δ-Amino-n-Valeriansäure sind und S₂ = N-hydroxiethylierte Purin- oder Pyrimindinbasen oder Zuckerrest-Purinoder Zuckerrest-Pyrimidinbase und F₄ - S₃ = Phospholipid mit z. B. S₃ = Cholin bedeuten.

Die Aufarbeitung der Polymerdispersionen erfolgt durch Sprühtrocknung, Fällung, Elektrophorese oder einen anderen Form der Trennung fest-flüssig mit anschließender Trocknung.

Das trockene Pulver wird allein oder in Abmischung mit VC-Homopolymerisaten (E-, S- oder Mikro-S-PVC) und/oder Copolymerisaten mit den für die jeweilige Verarbeitung notwendigen und für die spätere Verwendung zugelassenen Hilfsmitteln wie Stabilisatoren, Gleitsubstanzen, Weichmachern etc. entweder über eine Granulat-Zwischenstufe oder direkt zu Gegenständen für medizinische Anwendungen verarbeitet. Die Formmassen können mit 5 bis 85 Gew-% eines Weichmachers wie Monound Diester von Dicarbonsäuren und Hydroxy-Dicarbonsäuren, epoxidierte Naturöle, epoxidierte Fettsäuren und deren Ester, Polyester und Ester der Trimellinsäure abgemischt sein. Bevorzugt werden Dioctylphthalat, Zitronensäureester, epxodierte Soja- und Leinöle und Alkylepoxystearate mit Epoxidgehalten zwischen 1,5 und 10 % eingesetzt.

Diese Ausformung zu Gegenständen mit bioaktiven Oberflächen erfordert besondere Maßnahmen, wie ein einfaches Experiment verdeutlichen soll:

Stellt man z.B. ein Vinylchlorid-Polymer nach einem der oben beschriebenen Verfahren her, löst dieses in Tetrahydrofuran, gießt die Lösung in eine flache Petrischale und läßt das Lösungsmittel verdunsten, so erhält man eine Folie. Wird nun an dieser Folie mit Hilfe geeigneter analytischer Methoden senkrecht zur Oberfläche die Verteilung der Sulfonatfunktionen gemessen, so findet man überraschend eine inhomogene Verteilung, nämlich eine Verarmung an der von Luft kontaktierten Oberseite und eine Anreicherung an der dem Glas zugewendeten Unterseite.

Ähnliche Ergebnisse erhält man auch bei der Verarbeitung, z. B. der Extrusion zu Schläuchen für den Einsatz als Katheter. In diesem Fall sind beide Oberflächen, die Innen- und die Außenseite, an bioaktiven Funktionen verarmt.

Wenn die formgebenden Werkzeuge in den Bereichen, in denen mit beginnender Abkühlung die Ausformung der homogenen Schmelze zu Gegenständen stattfindet, mit polaren Oberflächen ausgerüstet sind, kommt man zu einem besonders guten Ergebnis: Die bioaktiven Funktionen sind gleichmäßig in den Innen- und Außenbezirken des Gegenstandes verteilt oder sogar an deren Grenzflächen angereichert.

Die Ausrüstung der Oberflächen der formgebenden Werkzeuge sollte an der Kontaktfläche eine Oberflächenspannung > 50 mN/m aufweisen und kann auf verschiedene Weisen erfolgen:
◆ Beschichtung oder Auskleidung mit wärmebeständigen Werkstoffen wie Metallegierungen oder Kunststoffen (vernetzt oder unvernetzt) wie z. B. Phenol/Formaldehyd-Kondensaten, Nylon-4, Nylon-6, Nylon-6,6 oder keramischen Stoffen
◆ Erzeugen eines kontinuierlichen Wasser/Wasserdampf-Gleitfilms.

Der Nachweis der Bioaktivität, speziell des antikoagulierenden Verhaltens solcher Gegenstände, z. B. von Schläuchen, wird wie folgt durchgeführt:

Die Schläuche werden in einer Umlaufapparatur (15 ml/min)
- 3 Waschzyklen zu 3 Stunden mit 1,5 molarer NaCl bei 90 °C
- 3 Waschzyklen zu 3 Stunden mit Wasser bei Raumtemperatur
- 3 Waschzyklen zu 3 Stunden mit Michaelis Puffer bei Raumtemperatur unterworfen.

Anschließend werden Absorptionsexperimente mit radioaktiv markiertem Human Serum Albumin, ¹²⁵I-HSA, durchgeführt, um die wirksame Oberfläche zu bestimmen. Dazu werden Schläuche wiederum in einer Umlaufapparatur (15 ml(min) bei Raumtemperatur 40 min lang einer ¹²⁵I-HSA Pufferlösung (3 mg/ml) ausgesetzt. Anschließend stellt man an den leeren Schläuchen die Radioaktivität fest. Die spezifische Menge an adsorbiertem Albumin in mg/cm² geometrischer Oberfläche ergibt sich dann aus der resultierenden Radioaktivität in cpm (counts per minute) dividiert durch die spezifische Radioaktivität des ¹²⁵I-HSA in cpm/mg, weiterhin dividiert durch die spezifische Oberfläche in cm².

Schließlich wird die Thrombin (T) - Antithrombin (AT)-Wechselwirkung in Gegenwart der Schlauchinnenflächen bestimmt. Als Maß für diese Wechselwirkung wird die Geschwindigkeitskonstante kₐₚₚ in ml/(u · min · 10⁻³) (u = units = Einheiten) in der Reaktion 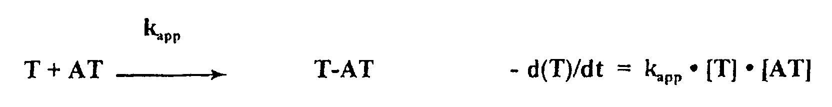 herangezogen.

Dazu wird AT in Michaelis Puffer (0,2 u/ml) in einer Umlaufapparatur (15 ml/min) während 30 min umgepumpt. Dann wird T (1.000 u/ml) in 0,25 ml-Inkrementen bis zu einem Gesamtvolumen von 5 ml zur zirkulierenden Lösung hinzugefügt. Die T-AT Reaktion wird nun nach Entnahme von 0,12 ml Lösung in 5 Minutenintervallen durch T-Bestimmung verfolgt. Trägt man 1/T gegen die Zeit auf, so erhält man eine Gerade mit der Steigung kₐₚₚ.

Aufgrund der beschriebenen vielseitigen Verfahren zur Herstellung von Formmassen und Gegenständen mit variabler Konzentration und Kombination bioaktiver Funktionen ergibt sich eine große Vielfalt von Anwendungsmöglichkeiten für die resultierenden Gegenstände.

Das erfindungsgemäß erhaltene Pulver kann sowohl in gelöster Form als auch in fester Form als Coatingmittel mit antikoagulierender Wirkung verwendet werden. Die zu Gegenständen ausgeformten Formmassen sind dank der einstellbaren bioaktiven Oberflächen für den medizinischen und chirurgischen Gebrauch im weitesten Sinne einsetzbar, z. B. als Schläuche, Katheter, Blutbeutel, Folien, Fäden, kardiovaskuläre Prothesen etc.

Ein weiteres Feld erschließt sich auch für die Verwendung als stationäre Phase, kompakt oder in dünner Schicht auf einem mechanisch stabilen Träger, in allen Arten chromatographischer Trennverfahren zur Reindarstellung bestimmter Blutbestandteile, Enzyme, Co-Enzyme oder enzymatischer Komplexe. Schließlich können die beanspruchten Systeme auch für verschiedene biologische Analysenverfahren Verwendung finden, z. B. bei der Elektrophorese, der Immunelektrophorese, RIA, ELISA usw.

Die folgende Beispiele beschreiben weitere Details der Erfindung.

### Beispiele

### Beispiel 1 (Emulsionspolymerisation)

In der ersten Stufe werden 88,7 Teile entsalztes Wasser (alle weiteren Angaben in Teile (Tle.) beziehen sich auf die gesamte organische Phase = 100 Tle.), 5 Teile Maleinsäuremonobutylester (MMBE) und 0,2 Tle. MARLON A315 (Alkylbenzolsulfonat, OXENO Olefinchemie GmbH) als 15 %ige Lösung in Wasser in einen beheizbaren 40-Liter-Rührreaktor mit Paddelrührer vorgelegt und auf 77 °C aufgeheizt. Anschließend werden folgende weitere Monomere innerhalb von 6 Stunden zudosiert:
5 Teile Natriumstyrolsulfonat (NaSS) als 10 %ige Lösung in Wasser
5 Teile Methylmethacrylat (MMA)
5 Teile Hydroxypropylacrylat (HPA)

In gleicher Weise wird eine 2 %ige wäßrige Initiatorlösung (Ammoniumperoxodisulfat, APDS), insgesamt 0,31 Teile, innerhalb von 8 Stunden zudosiert.

Nach Beendigung der Initiatorzugabe wird der Reaktorinhalt weitere 9 Stunden auf 77 °C gehalten.

In einer zweiten Stufe werden dann bei 53 °C 154,8 Teile entsalztes Wasser und 80 Teile Vinylchlorid (VC) zugesetzt. Dabei stellt sich ein Druck von etwa 6 bar ein.

Anschließend wird das folgende Initiatorsystem innerhalb von 3,5 Stunden zudosiert:
0,17 Teile Ammoniumperoxodisulfat als 2 %ige Lösung in Wasser
0,026 Teile Ascorbinsäure (AS) als 0,3 % ige Lösung in Wasser.

Eine zusätzliche Menge von 0,5 Teilen MARLON A315 wird gleichzeitig als 5 %ige Lösung in Wasser innerhalb von 3 Stunden zugegeben.

Nach Druckabfall wird die Polymerisation weitere 60 min fortgeführt. Anschließend wird die Dispersion aufRaumtemperatur heruntergekühlt, entgast und zu einem weißen Pulver sprühgetrocknet.

### Beispiel 2 (Lösungspolymerisation)

In diesem Experiment werden 50 Teile VC in 155,3 Tle. eines Ethanol-Wasser-Azeotrops gelöst und mit 1,6 Tle. Azobisisobutyronitril (AIBN) als Initiator vermischt. Nach dem Aufheizen auf 64 °C und dem Start der Reaktion (Beobachtung einer Wärmetönung), werden folgende Comonomere in 18 h zudosoiert:
18,75 Tle. Hydroxyethylmethacrylat (HEMA)
18,75 Tle. MMBE
12,5 Tle. NaSS.

Gleichzeitig gibt man 6,2 Tle. einer 5 %igen Lösung von AIBN in Ethanol innerhalb von 12 Stunden zu.

24 Stunden nach dem Ende der Initiatordosierung wird der Reaktor auf Raumtemperatur abgekühlt. Nach Entmonomerisierung wird die feine Copolymersuspension zu einem weißen Pulver sprühgetrocknet.

### Beispiel 3 (Emulsionspolymerisation)

In der ersten Stufe werden 81,4 Teile entsalztes Wasser, 18,75 Teile Maleinsäuremonobutylester (MMBE) und 0,2 Tle. MARLON A315 (Alkylbenzolsulfonat, OXENO Olefinchemie GmbH) als 15 %ige Lösung in Wasser in einen beheizbaren 40-Liter-Rührreaktor mit Paddelrührer vorgelegt und auf 77 °C aufgeheizt. Anschließend werden folgende weitere Monomere innerhalb von 6 Stunden zudosiert:
12,5 Teile Natriumstyrolsulfonat (NaSS) als 10 %ige Lösung in Wasser
18,75 Teile Hydroxyethylmethacrylat (HEMA)

In gleicher Weise wird eine 2 %ige wäßrige Initiatorlösung (Ammoniumperoxodisulfat, APDS), insgesamt 0,31 Teile, innerhalb von 6 Stunden zudosiert.

Nach Beendigung der Initiatorzugabe wird der Reaktorinhalt weitere 11 Stunden auf 77 °C gehalten.

In einer zweiten Stufe werden dann bei 53 °C 94,2 Teile entsalztes Wasser und 50 Teile Vinylchlorid (VC) zugesetzt. Dabei stellt sich ein Druck von etwa 6 bar ein. Anschließend wird das folgende Initiatorsystem innerhalb von 5,5 Stunden zudosiert:
0,31 Teile Ammoniumperoxodisulfat als 2 %ige Lösung in Wasser
0,046 Teile Ascorbinsäure (AS) als 0,3 %ige Lösung in Wasser.

Eine zusätzliche Menge von 0,5 Teilen MARLON A315 wird gleichzeitig als 5 %ige Lösung in Wasser innerhalb von 3 Stunden zugegeben.

Nach Druckabfall wird die Polymerisation weitere 60 min fortgeführt. Anschließend wird die Dispersion auf Raumtemperatur heruntergekühlt, entgast und zu einem weißen Pulver sprühgetrocknet.

### Beispiel 4 (Emulsionspolymerisation)

In der ersten Stufe werden 196,6 Teile entsalztes Wasser, 12,5 Teile Maleinsäuremonobutylester (MMBE) und 0,2 Tle. MARLON A315 (Alkylbenzolsulfonat, OXENO Olefinchemie GmbH) als 15 %ige Lösung in Wasser in einen beheizbaren 250-Liter-Rührreaktor mit Paddelrührer vorgelegt und auf 77 °C aufgeheizt. Anschließend werden folgende weitere Monomere innerhalb von 6 Stunden zudosiert:
12,5 Teile Natriumstyrolsulfonat (NaSS) als 10 %ige Lösung in Wasser
12,5 Teile Methylmethacrylat (MMA)
12,5 Teile Hydroxypropylacrylat (HPA)

In gleicher Weise wird eine 2 %ige wäßrige Initiatorlösung (Ammoniumperoxodisulfat, APDS), insgesamt 0,25 Teile, innerhalb von 8 Stunden zudosiert.

Nach Beendigung der Initiatorzugabe wird der Reaktorinhalt weitere 11 Stunden auf 77 °C gehalten.

In einer zweiten Stufe werden dann bei 53 °C 50 Teile Vinylchlorid (VC) zugesetzt. Dabei stellt sich ein Druck von etwa 6 bar ein. Anschließend wird das folgende Initiatorsystem innerhalb von 8 Stunden zudosiert:
0,51 Teile Ammoniumperoxodisulfat als 2 %ige Lösung in Wasser
0,034 Teile Ascorbinsäure (AS) als 0,3 %ige Lösung in Wasser.

Eine zusätzliche Menge von 0,5 Teilen MARLON A315 wird gleichzeitig als 5 %ige Lösung in Wasser innerhalb von 8 Stunden zugegeben.

Nach Druckabfall wird die Polymerisation weitere 60 min fortgeführt. Anschließend wird die Dispersion auf Raumtemperatur heruntergekühlt, entgast und zu einem weißen Pulver sprühgetrocknet.

Die Rezepturen der zuvor genannten Beispiele sind in Tabelle 1 zusammengefaßt:

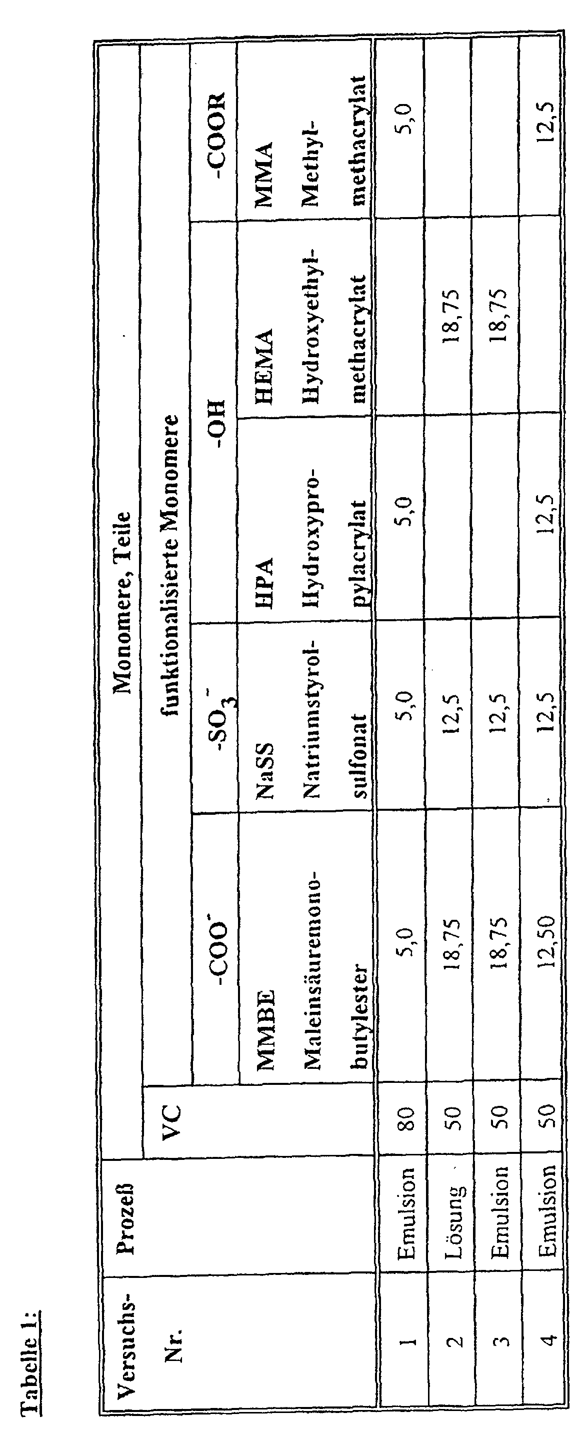

### Beispiel 5 (Verarbeitungsversuche)

In einem Laborextruder (GÖTTFERT MEX 30, Einschneckenextruder) werden Trocken-mischungen, hergestellt in einem temperierbaren Mischer, der in Tabelle 2 zusammengefaßten Rezepturen bei einem Temperaturgradienten zwischen 160 und 180 °C entlang der Extrusionsschnecke verarbeitet. Die biologischen Aktivitäten der resultierenden Schläuche sind ebenfalls in Tabelle 2 dargestellt.

**Tabelle 2:**

| **Einsatzstoffe** | **Schlauch 1** | **Schlauch 2** | **Schlauch 3** |
|---|---|---|---|
| S-PVC (a) | | 85 | 85 |
| Copolymer aus Beispiel 1 | 100 | | |
| Copolymer aus Beispiel 2 (b) | | 15 | |
| Copolymer aus Beispiel 3 | | | 15 |
| Copolymer aus Beispiel 4 | | | |
| EDENOL 1 B 35 (c) | 50 | 18,75 | 15 |
| VESTINOL AH (d) | 50 | 41,25 | 45 |
| Stabilisator (e) | 1 | 1 | 1 |
| Copolymeranteil im Compound (%) | 49,8 | 9,3 | 9,3 |
| Anteil -COOH-Monomer (%) im Compound | 2,49 | 1,75 | 1,75 |
| Anteil -SO₃Na-Monomer (%) im Compound | 2,49 | 1,16 | 1,16 |
| Anteil -OH-Monomer (%) im Compound | 2,49 | 1,75 | 1,75 |
| Biologische Aktivität kₐₚₚ (ml/u·min x 10⁻³) (f) | 4,75 | 4,85 | 5,4 |

| | | | |
|---|---|---|---|
| (a) Kommerzielles Suspensions-PVC für medizinische Ahwendungen (Solvay - SOLVIC 271 GA) | | | |
| (b) Copolymer, synthetisiert über Lösungspolymerisation | | | |
| (c) Epoxidiertes Alkylstearat, Epoxygehalt 4,5 bis 5,5 % | | | |
| (d) Dioctylphthalat (OXENO Olefinchemie GmbH) | | | |
| (e) STABIOL CZ 1616/6 (Calcium-/Zinkstearat, Henkel) | | | |
| (f) Geschwindigkeitskonstatne der Thrombin-Antithrombin-Rcaktion (T-AT Reaktion) | | | |

### Beispiel 6 (Verarbeitung mit gecoatetem Extrusionsdorn)

In einem Laborextruder (GÖTTFERT MEX 30, Einschneckenextruder) wird eine Mischung aus 100 Gewichtsteilen des aus Beispiel 1 erhaltenen Copolymeren mit 70 Gewichtsteilen EDENOL B 316 (Epoxidiertes Leinöl, Henkel), 30 Gewichtsteilen Dioctylphthalat und 1 Gewichtsteil Ca/Zn-Stearat zu Schläuchen mit 3 mm Innendurchmesser verarbeitet.

Die gewünschten bioaktiven Funktionen sind in noch größeren Mengen auf der Schlauchoberfläche nachzuweisen, wenn das formgebende Werkzeug mit einer polaren Oberfläche als nur V2A-Stahl ausgerüstet wird. Die Oberfläche des formgebenden Werkzeugs bestand in diesem Beispiel aus: 1. V2A-Stahl (als Vergleich), 2. Phenol-Formaldehydharz («polares coating»).

Die Tabellen 3 und 4 zeigen die unterschiedlichen chemischen und physikalischen Eigenschaften der Extrudate. Nach Verarbeitung über einen phenolharzgecoateten Extrusionsdorn findet man im Vergleich zur Extrusion mit reinem V2A-Stahl deutlich mehr Sauerstoff- und Schwefelatome an der Oberfläche und einen wesentlich höheren polaren Anteil an der Oberflächenspannung.

**Tabelle 3:**

| ESCA-Messungen an VC-Copolymer-Schläuchen aus Beispiel 6 | | | |
|---|---|---|---|
| **Element** | **Peak** | **VC-Copolymer über Metalldorn extrudiert (Atom-%)** | **VC-Copolymer über Phenolharzdorn extrudiert (Atom-%)** |
| C | 1 s | 76,5 | 66,6 |
| O | 1 s | 11,5 | 19,9 |
| Cl | 2 p | 11,0 | 6,7 |
| Na | 1 s | 0,4 | 4,9 |
| S | 2 p | 0,3 | 2,8 |

**Tabelle 4:**

| Messungen der Oberflächenspannung an VC-Copolymer-Schläuchen aus Beispiel 6 | | | | | |
|---|---|---|---|---|---|
| **Proben Nr.** | **Kontaktwinkel** | | **Oberflächenspannung** | | |
| | **H**_{**2**}**O** **[grd]** | **CH**_{**2**}**I**_{**2**} **[grd]** | **disperser Anteil [mN/m]** | **polarer Anteil** **[mN/m]** | **Summe** **[mN/m]** |
| VC-Copolymer über Metalldorn extrudiert | 91 | 35 | 41,5 | 0,6 | 42,1 |
| VC-Copolymer über Phenolharzdorn extrudiert | 64 | 60 | 22,4 | 18,0 | 40,4 |

## Patentansprüche

1. Verwendung von Copolymeren, die durch radikalische Polymerisation von hydrophoben und hydrophilen, ganz oder teilweise mit biospezifisch wirksamen Funktionen ausgestatteten Monomeren der allgemeinen Formeln
M-F₁
M-F₂
M-F₃
M-F₁-F₄
wobei
M = Rest des hydrophilen Monomeren und
F₁ = Alkyl-, Aryl- oder Alkyl/Arylhydroxyl-
F₂ = Alkyl-, Aryl- oder Alkyl/Arylcarboxyl-
F₃ = Alkyl-, Aryl- oder Alkyl/Arylsulfonyl-
F₄ = Alkyl-, Aryl- oder Alkyl/Arylphosphatyl-
hergestellt sind, zur Herstellung von Formmassen mit antikoagulierenden Eigenschaften.

2. Verwendung der Copolymeren nach Anspruch 1,
**dadurch gekennzeichnet,**
**dass** als hydrophobe Monomere Styrol, Methylmethacrylat und Vinylchlorid eingesetzt werden.

3. Verwendung der Copolymeren nach zumindest einem der vorherigen Ansprüche,
**dadurch gekennzeichnet,**
**dass** als hydrophile Monomere Styrol- und/oder (Meth-)Acryl- und/oder Maleinyl- und/oder Vinylether-Verbindungen eingesetzt werden.

4. Verwendung der Copolymeren nach zumindest einem der vorherigen Ansprüche,
**dadurch gekennzeichnet,**
**dass** als hydrophile Monomere Styrolsulfonat, Hydroxyalkyl-(meth)acrylat, Methacrylsäure, Maleinsäuremonoester, Vinyl-(EO)ₙ-Ether eingesetzt werden.

5. Verwendung der Copolymeren nach zumindest einem der vorherigen Ansprüche,
**dadurch gekennzeichnet,**
**dass** die Monomeren in Dispersion oder in Lösung radikalisch copolymerisiert werden, und dass als Dispersionsmedium bzw. als Lösungsmittel Alkylalkohole, Wasser, Ketone und Ether, einzeln oder gemischt, Verwendung finden.

6. Verwendung der Copolymeren nach zumindest einem der vorherigen Ansprüche,
**dadurch gekennzeichnet,**
**dass** die hydrophilen, ganz oder teilweise mit biospezifisch wirksamer Funktion ausgestatteten Monomere ausgewählt sind aus solchen, die den Formeln entsprechen
M-F₂-S₁
M-F₃-S₁
M-F₁-F₄-S₁
M-F₁-F₄-S₂
M-F₁-F₄-S₃
Mit S₁ = Rest einer Aminosäure, die über -NH₂ an Carboxyl-, Sulfonyl- oder Phosphatyl- gebunden ist, wobei die bevorzugten Aminosäuren Glutamin, Threonin, Methionin, Cystein, Cysteinsäure, Prolin, Hydroxyprolin, Serin, Tyrosin, Benzoyloxycarbonyl-Lysin, tert.-Butyloxycarbonyl-Lysin, ε-Aminocapronsäure, β-Alanin, γ-Amino-n-Buttersäure und substituierte oder unsubstituierte δ-Amino-n-Valeriansäure sind und S₂ = Rest einer N-hydroxyethylierte n Purin- oder Pyrimidinbase oder Zuckerrest-Purin- oder Zuckerrest-Pyrimidinbase und F₄ - S₃ = Phospholipid mit S₃ = Cholin bedeuten.

7. Verwendung der Copolymeren nach zumindest einem der vorherigen Ansprüche in Abmischung mit VC-Homo- und/oder Copolymerisaten.

8. Verwendung der Copolymeren nach zumindest einem der vorherigen Ansprüche in Abmischung mit 5 bis 85 Gew.-% eines Weichmachers.

9. Verwendung der Copolymeren nach zumindest einem der vorherigen Ansprüche,
**dadurch gekennzeichnet,**
**dass** als Weichmacher Mono- und Diester von Dicarbonsäuren und Hydroxy-Dicarbonsäuren, epoxidierte Naturöle, epoxidierte Fettsäuren und deren Ester, Polyester, und Ester der Trimellinsäure eingesetzt werden.

10. Verwendung der Copolymeren nach zumindest einem der vorherigen Ansprüche in Abmischung mit den für die jeweilige Verarbeitung und für die spätere Verwendung zugelassenen Hilfsmittel wie Stabilisatoren, Gleitsubstanzen, Farbstoffen und Pigmenten.

11. Verwendung der Formmassen nach zumindest einem der vorherigen Ansprüche zur kontinuierlichen oder diskontinuierlichen Ausformung der nach zumindest einem der vorherigen Ansprüche hergestellten Formmassen zu Gegenständen für medizinische Anwendungen, **dadurch gekennzeichnet, dass** die mit der homogenen Schmelze im Kontakt stehenden formgebenden Werkzeuge an dieser Kontaktfläche eine Oberflächenspannung > 50 mN/m aufweisen.

12. Verwendung der Formmassen nach zumindest einem der vorherigen Ansprüche,
**dadurch gekennzeichnet,**
**dass** diese Oberflächenspannung bei der Extrusion oder Coextrusion durch eine geeignete Metallegierung, durch einen Wasser-/Wasserdampf-Gleitfilm oder durch einen Polymerbelag aus Nylon-4, Nylon-6, Nylon-6,6 oder ein vernetztes Phenol-Formaldehydharz oder durch eine Keramik-Auflage eingestellt wird.

13. Verwendung der Formmassen nach zumindest einem der vorherigen Ansprüche als Arzneimittel mit antikoagulierender Wirkung.

14. Verwendung der Formmassen nach zumindest einem der vorherigen Ansprüche und der Gegenstände nach zumindest einem der vorherigen Ansprüche für den medizinischen und chirurgischen Gebrauch, für Rohre, Katheter, Folien oder Fäden und cardiovaskuläre Prothesen.

15. Verwendung der Formmassen nach zumindest einem der vorherigen Ansprüche und der Gegenstände nach zumindest einem der vorherigen Ansprüche als stationäre Phase, kompakt oder in dünner Schicht auf einem mechanisch stabilen Träger, in der Ionenaustausch- und Affinitätschromatographie.

16. Verwendung der Formmassen nach zumindest einem der vorherigen Ansprüche und der Gegenstände nach zumindest einem der vorherigen Ansprüche zur selektiven Abtrennung und Reindarstellung bestimmter Bestandteile des Blutplasmas.

17. Verwendung der Formmassen nach zumindest einem der vorherigen Ansprüche und der Gegenstände nach zumindest einem der vorherigen Ansprüche zur selektiven Abtrennung und Reindarstellung von Enzymen, Co-Enzymen oder enzymatischen Komplexen.

18. Verwendung der Formmassen nach zumindest einem der vorherigen Ansprüche und der Gegenstände nach zumindest einem der vorherigen Ansprüche in Meßsystemen für die biologische Analyse.

## Claims

1. Use of copolymers that are prepared by radical polymerization of hydrophobic monomers and hydrophilic monomers that entirely or partially bear biospecifically active functional groups of the general formulae:
M-F₁
M-F₂
M-F₃
M-F₁-F₄
wherein:
M is a radical of the hydrophilic monomer and
F₁ is an alkyl, aryl or alkyl/arylhydroxyl group,
F₂ is an alkyl, aryl or alkyl/arylcarboxyl group,
F₃ is an alkyl, aryl or alkyl/arylsulfonyl group,
F₄ is an alkyl, aryl or alkyl/arylphosphatyl group,
for the preparation of molding materials having anticoagulant properties.

2. Use of the copolymers according to claim 1,
**characterized in that**,
styrene, methylmethacrylate and vinylchloride are used as hydrophobic monomers.

3. Use of the copolymers according to at least one of the preceding claims,
**characterized in that**,
styrene and/or (meth)acryl and/or maleinyl and/or vinylether compounds are used as hydrophilic monomers.

4. Use of the copolymers according to at least one of the preceding claims,
**characterized in that**,
styrene sulfonate, hydroxyalkyl-(meth)acrylate, methacrylic acid, maleic acid monoesters, vinyl-(E0)ₙ-ethers are used as hydrophilic monomers.

5. Use of the copolymers according to at least one of the preceding claims,
**characterized in that**,
the monomers are radically copolymerized in dispersion or in solution using alkyl alcohols, water, ketones and ethers, alone or in admixture, as dispersion medium and solvent, respectively.

6. Use of the copolymers according to at least one of the preceding claims,
**characterized in that**,
the hydrophilic monomers entirely or partially bearing biospecifically active functional groups are selected from compounds corresponding to the formulae:
M-F₁-S₁
M-F₃-S₁
M-F₁-F₄-S₁
M-F₁-F₄-S₂
M-F₁-F₄-S₃
wherein S₁ is a radical of an amino acid which is bonded via -NH₂ to the carboxyl, sulfonyl or phosphatyl group whereby the preferred amino acids are glutamine, threonine, methionine, cysteine, cysteic acid, proline, hydroxyproline, serine, tyrosine, benzoyloxycarbonyl-lysine, tert-butyloxycarbonyl-lysine, ε-aminocaproic acid, β-alanine, γ-amino-n-butyric acid, and substituted or unsubstituted δ-amino-n-valeric acid, and S₂ is a radical of N-hydroxyethylated purine or pyrimidine bases, or sugar radical purine or sugar radical pyrimidin base, and F₄-S₃ is phospholipid wherein S₃ is choline.

7. Use of the copolymers according to at least one of the preceding claims in admixture with VC-homo and/or copolymerisates.

8. Use of the copolymers according to at least one of the preceding claims in admixture with 5 to 85 wt% of a plasticizer.

9. Use of the copolymers according to at least one of the preceding claims,
**characterized in that**,
mono- and diesters of dicarboxylic acids and hydroxy dicarboxylic acids, epoxidized natural oils, epoxidized fatty acids and their esters, polyesters and esters of trimellitic acid are used as plasticizers.

10. Use of the copolymers according to at least one of the preceding claims in admixture with auxiliaries, like stabilizers, lubricants, dyes and pigments, that are approved for the respective processing and the subsequent use.

11. Use of the molding materials according to at least one of the preceding claims for the continuous or discontinuous molding of the molding material obtained according to at least one of the preceding claims to obtain articles for medical use,
**characterized in that**,
the shaping molds in contact with the homogeneous melt have a surface tension at said contacting surface of >50 mN/m.

12. Use of the molding materials according to at least one of the preceding claims,
**characterized in that**,
said surface tension during extrusion or coextrusion is adjusted by a suitable metal alloy, by a water/steam gliding film or by a polymer coating comprised of nylon-4, nylon-6, nylon-6,6 or a crosslinked phenol formaldehyde resin or by a ceramic coating.

13. Use of the molding materials according to at least one of the preceding claims as pharmaceutical with anticoagulant function.

14. Use of the molding materials according to at least one of the preceding claims and the articles according to at least one of the preceding claims for medical or surgical use, for tubes, catheters, films or threads and cardiovascular prostheses.

15. Use of the molding materials according to at least one of the preceding claims and the articles according to at least one of the preceding claims as stationary phase in compact form or in a thin layer on a mechanically stable substrate, in ionic exchange and affinity chromatography.

16. Use of the molding materials according to at least one of the preceding claims and the articles according to at least one of the preceding claims for the selective separation and pure preparation of specific ingredients of blood plasma.

17. Use of the molding materials according to at least one of the preceding claims and the articles according to at least one of the preceding claims for the selective separation and pure preparation of enzymes, co-enzymes or enzymatic complexes.

18. Use of the molding materials according to at least one of the preceding claims and the articles according to at least one of the preceding claims in measuring systems for the biological analysis.

## Revendications

1. Utilisation de copolymères, qui sont préparés par polymérisation radicalaire de monomères hydrophobes et hydrophiles, pourvus totalement ou partiellement de fonctions biospécifiquement actives, de formules générales
M - F₁
M - F₂
M - F₃
M - F₁ - F₄
où M = radical du monomère hydrophile et
F₁ = un alkyle, un aryle ou un alkyl/arylhydroxyle,
F₂ = un alkyle, un aryle ou un alkyl/arylcarboxyle,
F₃ = un alkyle, un aryle ou un alkyl/arylsulfonyle,
F₄ = un alkyle, un aryle ou un alkyl/arylphosphatyle
pour la préparation de masses de moulage avec des propriétés anticoagulantes.

2. Utilisation des copolymères selon la revendication 1, **caractérisée en ce qu'**on utilise comme monomères hydrophobes, du styrène, du méthacrylate de méthyle et du chlorure de vinyle.

3. Utilisation des copolymères selon au moins l'une quelconque des revendications précédentes, **caractérisée en ce qu'**on utilise comme monomères hydrophiles des composés de styrène et/ou de (méth)acryle et/ou de maléinyle et/ou de vinyléther.

4. Utilisation des copolymères selon au moins l'une quelconque des revendications précédentes, **caractérisée en ce qu'**on utilise comme monomères hydrophiles du sulfonate de styrène, du (méth)acrylate d'hydroxyalkyle, de l'acide méthacrylique, des monoesters de l'acide maléique, des vinyl-(OE)ₙ-éthers.

5. Utilisation des copolymères selon au moins l'une quelconque des revendications précédentes, **caractérisée en ce que** les monomères sont copolymérisés de manière radicalaire dans une dispersion ou une solution et **en ce qu'**on utilise comme milieu de dispersion ou, selon le cas, comme solvant, des alcools alkyliques, de l'eau, des cétones et des éthers, seuls ou en mélange.

6. Utilisation des copolymères selon au moins l'une quelconque des revendications précédentes, **caractérisée en ce que** les monomères hydrophiles, totalement ou partiellement munis de fonction biospécifiquement active sont choisis parmi ceux qui correspondent aux formules :
M - F₂ - S₁
M - F₃ - S₁
M - F₁ - F₄ - S₁
M - F₁ - F₄ - S₂
M - F₁ - F₄ - S₃
avec S₁ = un radical d'un acide aminé, qui est lié via -NH₂ à un carboxyle, sulfonyle ou phosphatyle, les acides aminés préférés étant la glutamine, la thréonine, la méthionine, la cystéine, l'acide cystéique, la proline, l'hydroxyproline, la sérine, la tyrosine, la benzoyloxycarbonyllysine, la tert-butyloxycarbonyl-lysine, l'acide ε-aminocaprique, la β-alanine, l'acide γ-amino-n-butyrique et l'acide δ-amino-n-valérique substitué ou non substitué et S₂ = radical d'une base de purine ou de pyrimidine N-hydroxyéthylées ou des bases (radical de sucre)-purine ou (radical de sucre)-pyrimidine et F₄ - S₃ = un phospholipide avec S₃ = de la choline.

7. Utilisation des copolymères selon au moins l'une quelconque des revendications précédentes en mélange avec des homopolymères et/ou des copolymères de CV.

8. Utilisation des copolymères selon au moins l'une quelconque des revendications précédentes, en mélange avec 5 à 85% en poids d'un plastifiant.

9. Utilisation des copolymères selon au moins l'une quelconque des revendications précédentes, **caractérisée en ce qu'**on utilise comme plastifiant des monoesters et des diesters d'acides dicarboxyliques et d'acides hydroxydicarboxyliques, des huiles naturelles époxydées, des acides gras époxydés et leurs esters, des polyesters et des esters de l'acide trimellitique.

10. Utilisation des copolymères selon au moins l'une quelconque des revendications précédentes, en mélange avec les adjuvants autorisés pour chaque transformation et pour l'utilisation ultérieure, tels que des stabilisateurs, des lubrifiants, des colorants et des pigments.

11. Utilisation des masses de moulage selon au moins l'une quelconque des revendications précédentes, pour le moulage continu ou discontinu des masses de moulage préparées selon au moins l'une quelconque des revendications précédentes en objets pour des applications médicales, **caractérisée en ce que** les outils de moulage en contact avec les masses fondues homogènes présentent à cette surface de contact une tension superficielle > 50 mN/m.

12. Utilisation des masses de moulage selon au moins l'une quelconque des revendications précédentes, **caractérisée en ce que** cette tension superficielle lors de l'extrusion ou de la coextrusion est réglée par un alliage métallique approprié, par un film lubrifiant eau/vapeur d'eau ou par un revêtement polymère en Nylon-4, Nylon-6, Nylon-6,6 ou par une résine réticulée de phénol et de formaldéhyde ou par un revêtement en céramique.

13. Utilisation des masses de moulage selon au moins l'une quelconque des revendications précédentes, comme médicament avec une action anticoagulante.

14. Utilisation des masses de moulage selon au moins l'une quelconque des revendications précédentes et des objets selon au moins l'une quelconque des revendications précédentes pour l'utilisation médicale et chirurgicale, pour des tuyaux, des cathéters, des films ou des fils et des prothèses cardio-vasculaires.

15. Utilisation des masses de moulage selon au moins l'une quelconque des revendications précédentes et des objets selon au moins l'une quelconque des revendications précédentes comme phase stationnaire, compacte ou en couche mince sur un support mécaniquement stable, dans la chromatographie par échange ionique ou par affinité.

16. Utilisation des masses de moulage selon au moins l'une quelconque des revendications précédentes et des objets selon au moins l'une quelconque des revendications précédentes pour la séparation sélective et la préparation pure de constituants définis du plasma sanguin.

17. Utilisation des masses de moulage selon au moins l'une quelconque des revendications précédentes et des objets selon au moins l'une quelconque des revendications précédentes pour la séparation sélective et la préparation pure d'enzymes, de coenzymes ou de complexes enzymatiques.

18. Utilisation des masses de moulage selon au moins l'une quelconque des revendications précédentes et des objets selon au moins l'une quelconque des revendications précédentes dans des systèmes de mesure pour l'analyse biologique.
